(19) Europäisches Patentamt
European Patent Office
Office européen des brevets

(11) EP 3 928 625 A1

(12) **EUROPEAN PATENT APPLICATION**
published in accordance with Art. 153(4) EPC

(43) Date of publication:
**29.12.2021 Bulletin 2021/52**

(21) Application number: **20758918.5**

(22) Date of filing: **03.02.2020**

(51) Int Cl.:
***A23C 9/123*** (2006.01)          ***A23C 9/13*** (2006.01)
***C12N 1/20*** (2006.01)

(86) International application number:
**PCT/JP2020/003848**

(87) International publication number:
**WO 2020/170776 (27.08.2020 Gazette 2020/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.02.2019 JP 2019027288**

(71) Applicant: **Kabushiki Kaisha Yakult Honsha Tokyo, 105-8660 (JP)**

(72) Inventors:
• **SAITO, Junki**
  **Tokyo 105-8660 (JP)**
• **HOSHI, Ryotaro**
  **Tokyo 105-8660 (JP)**

(74) Representative: **Blodig, Wolfgang**
  **Wächtershäuser & Hartz**
  **Patentanwaltspartnerschaft mbB**
  **Weinstrasse 8**
  **80333 München (DE)**

(54) **METHOD FOR PRODUCING CULTURE OF LACTIC ACID BACTERIUM AND/OR BACTERIUM BELONGING TO GENUS BIFIDOBACTERIUM**

(57)    By a method for producing a culture for obtaining a culture by culturing a lactic acid bacterium and/or a bacterium belonging to the genus *Bifidobacterium* in a milk peptide-containing medium which is characterized in that a culture temperature is 3°C or more lower than an optimum culture temperature of the lactic acid bacterium and/or the bacterium belonging to the genus *Bifido-* *bacterium,* the change in the acidity during the storage of the product is not accelerated, and the bacterial count at the time of the preparation of the product can be maintained at a high level, even when the lactic acid bacterium or the like is cultured in a medium containing a milk peptide added thereto.

EP 3 928 625 A1

**Description**

Technical Field

The present invention relates to a method for producing a culture of a lactic acid bacterium and/or a bacterium belonging to the genus *Bifidobacterium* and to a culture produced by the method.

Background Art

It has been known that, when fermented milk is produced using a culture of a lactic acid bacterium and/or a bacterium belonging to the genus *Bifidobacterium,* addition of a milk peptide as an auxiliary culture agent to a medium can accelerate the growth of the lactic acid bacterium or the like, increase the initial bacterial count and shorten the fermentation period.

Addition of a milk peptide, however, has caused problems such as deterioration of the quality of the product due to a considerable increase in the change in the acidity during the storage of the product and the difficulty in maintaining the bacterial count at the time of the preparation of the product at a high level as a result of the increase in the acidity during the storage of the product.

As a method for inhibiting the increase in the acidity of fermented milk during the storage of the product, a method of adding a monoculture solution of a lactic acid bacterium belonging to the genus *Lactobacillus* to a lactic acid bacterium starter (a mixture starter) has been applied for the present. However, the effects are not sufficient, and it is shown in the application that the increase in the acidity was not inhibited when a milk peptide alone was added (PTL 1).

Citation List

Patent Literature

PTL 1: WO2013/133313

Summary of Invention

Technical Problem

Accordingly, an object of the present invention is to provide a technique with which the change in the acidity during the storage of the product is not accelerated and with which the bacterial count at the time of the preparation of the product can be maintained at a high level even when a lactic acid bacterium or the like is cultured in a medium containing a milk peptide added thereto.

Solution to Problem

As a result of intensive study to achieve the above-mentioned object, the present inventors have found that the object can be achieved by culturing a lactic acid bacterium or the like in a medium containing a milk peptide added thereto unexpectedly at a temperature which is 3°C or more lower than the optimum culture temperature of the lactic acid bacterium or the like, even though culturing is generally conducted at the optimum culture temperature. The present invention has been thus completed.

That is, the present invention relates to a method for producing a culture for obtaining a culture by culturing a lactic acid bacterium and/or a bacterium belonging to the genus *Bifidobacterium* in a milk peptide-containing medium which is characterized in that a culture temperature is 3°C or more lower than an optimum culture temperature of the lactic acid bacterium and/or the bacterium belonging to the genus *Bifidobacterium.*

Moreover, the present invention relates to a fermented food or drink characterized by containing a culture produced by the above-mentioned method for producing a culture.

Furthermore, the present invention relates to a culture produced by the above-mentioned method for producing a culture

which is characterized in that the acidity of the culture before storage is 4 to 12 and that the change in the acidity after storage at 10°C for 21 days is smaller than 1.45.

Moreover, the present invention relates to a culture produced by the above-mentioned method for producing a culture

which is characterized in that the viable cell count of the culture before storage is $1.2 \times 10^9$ cfu/ml or more and that the viability after storage at 10°C for 21 days is 80% or more.

Furthermore, the present invention relates to a fermented food or drink characterized by containing the above-mentioned culture.

Advantageous Effects of Invention

Regarding the method for producing a culture of the present invention, by a simple method of culturing at a temperature which is 3°C or more lower than the optimum culture temperature of a lactic acid bacterium and/or a bacterium belonging to the genus *Bifidobacterium* in a milk peptide-containing medium, a culture in which the change in the acidity during storage is inhibited and in which the viability of the lactic acid bacterium and/or the bacterium belonging to the genus *Bifidobacterium* contained therein is high can be obtained.

Moreover, a culture produced by the method for producing a culture of the present invention has the characteristics described above and thus can be used for the production of a fermented food or drink which can maintain the quality of the product also during storage.

In general, in a method for producing a culture for obtaining a culture by culturing a lactic acid bacterium and/or a bacterium belonging to the genus *Bifidobacterium,* it is the common technical knowledge that culturing is conducted at the optimum culture temperature, which is considered to most increase the bacterial count, and culturing at a different temperature is not generally conducted. When an auxiliary culture agent such as a milk peptide is added to the medium, it is expected that culturing at a temperature other than the optimum culture temperature would only increase (recover) the growth speed and consequently increase (or further deteriorate in some cases) the acidity, and the effects of the present invention (both recovery of the growth speed and inhibition of the increase in the acidity) would not be easily expected.

Description of Embodiments

The method for producing a culture of the present invention (hereinafter referred to as "the method of the present invention") is a method for producing a culture for obtaining a culture by culturing a lactic acid bacterium and/or a bacterium belonging to the genus *Bifidobacterium* (sometimes referred to as "a lactic acid bacterium or the like" below) in a milk peptide-containing medium in which the culture temperature is 3°C or more lower, preferably 5°C or more lower, more preferably 5 to 15°C lower than the optimum culture temperature of the lactic acid bacterium and/or the bacterium belonging to the genus *Bifidobacterium.* In the method of the present invention, when two or more lactic acid bacteria or the like having different optimum culture temperatures are used, the optimum culture temperature of any of the lactic acid bacteria may be used as a basis. For example, the optimum culture temperature of a lactic acid bacterium for which the effects of the present invention (inhibition of the change in the acidity and improvement of the viability) are to be obtained, namely a lactic acid bacterium which clearly contributes to the change in the acidity of a fermented food or drink or a lactic acid bacterium whose viability is to be improved in terms of the usefulness as a probiotic, may be used as a basis. Alternatively, in order to obtain the effects of the present invention (inhibition of the change in the acidity and improvement of the viability) for all the lactic acid bacteria or the like, the optimum culture temperature of a lactic acid bacterium or the like which has the lowest optimum culture temperature may be used as a basis.

The medium used in the method of the present invention may be a medium in which the lactic acid bacterium or the like can grow and which contains a milk peptide. Examples of such a medium include a mammalian milk medium composed of raw milk such as cow's milk, goat's milk, mare milk and sheep's milk, a dairy product such as defatted milk powder, whole milk powder and fresh cream or the like and a synthetic medium. Of the media, defatted milk powder is preferable.

The milk peptide contained in the medium is not particularly limited, and examples include those obtained from casein, whey or the like through treatment such as hydrolysis and the like. The molecular weight of the milk peptide is not particularly limited, either. The milk peptide content of the medium is not particularly limited and is for example 0.025 to 0.25 mass%, preferably 0.05 to 0.10 mass%.

Depending on the need, the medium may contain a saccharide which the lactic acid bacterium or the like can assimilate. Examples of such a saccharide include glucose, fructose, mannose, galactose, arabinose, ribose, xylose, lactose, maltose, sucrose, palatinose, trehalose, raffinose and the like. The amount of the saccharide which the lactic acid bacterium or the like can assimilate contained in the medium is not particularly limited and may be appropriately set depending on the lactic acid bacterium or the like used for culturing, and the amount is for example 0 to 20 mass%, preferably 2 to 8 mass%.

The medium preferably further contains an auxiliary culture agent such as a Chinese sweet tea extract, an oolong tea extract, a green tea extract, a black tea extract, a jasmine tea extract and oleic acid or the like because the viable cell count of the culture before storage becomes high. One or more kinds of the auxiliary culture agents may be used.

Regarding the Chinese sweet tea extract, the oolong tea extract, the green tea extract, the black tea extract and the jasmine tea extract of the auxiliary culture agents, those which are commercially available and those obtained by the methods described in JP-A-2001-178413, JP-A-2016-174589 and the like may be used. The extract content of the medium is not particularly limited, and for example, the Chinese sweet tea extract or oolong tea extract content is 0.1 to 2 mass%, preferably 0.22 to 0.33 mass%.

Of the auxiliary culture agents, oleic acid or the like is not particularly limited, and in addition to free oleic acid and inorganic salts of oleic acid, sugar esters, glycerides, sorbitan esters, propylene glycol esters and the like which are generally used as emulsifiers and in which the fatty acid moiety is oleic acid can be used. A food material which contains a large amount of oleic acid or the like can also be used.

Specific examples of oleic acid or the like include salts of oleic acid such as sodium oleate and potassium oleate and oleate esters such as glyceryl oleate, polyglyceryl oleate and sucrose oleate. One or more kinds of oleic acid and the like can be used.

The amount of oleic acid or the like contained in the medium is not particularly limited and is for example 5 to 200 ppm, preferably 10 to 100 ppm.

The medium may further contain a component which can be added to a general medium that is used for culturing a lactic acid bacterium or the like, such as vitamins including vitamin A, vitamin B's, vitamin C, vitamin E and the like, peptides other than milk peptides, amino acids and salts of calcium, magnesium and the like.

A preferable embodiment of the milk peptide-containing medium used in the method of the present invention is as follows.

| Milk peptide | 0.025 to 0.25 mass% |
|---|---|
| Defatted milk powder | 10 to 20 mass% |
| Glucose | 0 to 8 mass% |

The lactic acid bacterium or the like which is cultured in the milk peptide-containing medium in the method of the present invention is not particularly limited and for example may be any of those found in the nature, those deposited at depositary authorities, those which are commercially available and mutants thereof.

Examples of lactic acid bacteria include bacteria belonging to the genus *Lactobacillus* such as *Lactobacillus casei, Lactobacillus gasseri, Lactobacillus acidophilus, Lactobacillus cremoris, Lactobacillus helveticus, Lactobacillus salivarius, Lactobacillus fermentum, Lactobacillus jugurti, Lactobacillus delbrueckii* subsp. *bulgaricus, Lactobacillus delbrueckii* subsp. *delbrueckii* and *Lactobacillus johnsonii;* bacteria belonging to the genus *Streptococcus* such as *Streptococcus thermophilus;* bacteria belonging to the genus *Lactococcus* such as *Lactococcus lactis* subsp. *lactis, Lactococcus lactis* subsp. *cremoris, Lactococcus plantarum* and *Lactococcus raffinolactis* and bacteria belonging to the genus *Enterococcus* such as *Enterococcus faecalis* and *Enterococcus faecium.* One or more kinds of the lactic acid bacteria may be used.

Of the lactic acid bacteria, one or more kinds of lactic acid bacterium selected from the group consisting of a bacterium belonging to the genus *Lactobacillus,* a bacterium belonging to the genus *Streptococcus* and a bacterium belonging to the genus *Lactococcus* are preferable. One or more kinds of lactic acid bacterium selected from the group consisting of *Lactobacillus casei, Lactococcus lactis* and *Streptococcus thermophilus* are more preferable, and one or more kinds of lactic acid bacterium selected from the group consisting of *Lactobacillus casei* YIT9029 (FERM BP-1366, deposition date of January 12, 1981, optimum culture temperature of 37°C), *Lactococcus lactis* YIT2027 (FERM BP-6224, deposition date of February 10, 1997, optimum culture temperature of 30°C) and *Streptococcus thermophilus* YIT2021 (FERM BP-7537, deposition date of November 1, 1996, optimum culture temperature of 37°C) are particularly preferable. The lactic acid bacteria may also be used in combination with one or more kinds of the bacteria belonging to the genus *Bifidobacterium* below.

Examples of bacteria belonging to the genus *Bifidobacterium* include *Bifidobacterium breve, Bifidobacterium bifidum, Bifidobacterium longum, Bifidobacterium infantis, Bifidobacterium adolescentis, Bifidobacterium catenulatum, Bifidobacterium pseudocatenulatum, Bifidobacterium angulatum, Bifidobacterium gallicum, Bifidobacterium lactis, Bifidobacterium animalis* and the like. Of the bacteria belonging to the genus *Bifidobacterium, Bifidobacterium breve* is preferable. One or more kinds of the bacteria belonging to the genus *Bifidobacterium* may be used. The bacteria belonging to the genus *Bifidobacterium* may also be used in combination with one or more kinds of the lactic acid bacteria above.

Of the bacteria belonging to the genus *Bifidobacterium, Bifidobacterium breve* is more preferable, and *Bifidobacterium breve* YIT12272 (FERM BP-11320, deposition date of January 20, 2011, optimum culture temperature of 37°C) is particularly preferable. The bacteria belonging to the genus *Bifidobacterium* may also be used in combination with one or more kinds of the lactic acid bacteria above.

In the method of the present invention, a lactic acid bacterium and/or a bacterium belonging to the genus *Bifidobacterium* is used, and *Lactobacillus casei* and/or *Bifidobacterium breve* is preferable.

The lactic acid bacteria and the bacterium belonging to the genus *Bifidobacterium* which are described above with the deposition dates have all been deposited at International Patent Organism Depositary, National Institute of Technology and Evaluation (#120, 2-5-8 Kazusakamatari, Kisarazu-shi, Chiba 292-0818, Japan).

In the method of the present invention, the conditions for culturing the lactic acid bacterium or the like in the milk peptide-containing medium may be in accordance with the kind of the lactic acid bacterium or the like used, except that culturing is conducted at a temperature which is 3°C or more lower than the optimum culture temperature, and the conditions are not particularly limited.

Specifically, when a lactic acid bacterium is cultured in a milk peptide-containing medium, the conditions are that the lactic acid bacterium is inoculated in such a manner that the bacterial count of the medium becomes around $5 \times 10^6$ cfu/ml and that the bacterium is cultured at a temperature which is 3°C or more lower than the optimum culture temperature of the lactic acid bacterium or the like until the pH becomes around 3.6 or until the acidity becomes around 24. Regarding the culturing conditions here, a method which is suitable for culturing the lactic acid bacterium used may be appropriately selected from static method, stirring, shaking, aeration and the like.

When a bacterium belonging to the genus *Bifidobacterium* is cultured in a milk peptide-containing medium, the conditions are that the bacterium is inoculated in such a manner that the bacterial count of the medium becomes around $5 \times 10^6$ cfu/ml and that the bacterium is cultured at a temperature which is 3°C or more lower than the optimum culture temperature of the lactic acid bacterium or the like until the pH becomes around 4.9. Regarding the culturing conditions here, a method which is suitable for culturing the bacterium belonging to the genus *Bifidobacterium* used may be appropriately selected from static method, stirring, shaking and the like, and the conditions may also be anaerobic conditions.

When a lactic acid bacterium and a bacterium belonging to the genus *Bifidobacterium* are both cultured in a milk peptide-containing medium, the conditions are that the bacteria are inoculated in such a manner that the bacterial count of the bacterium belonging to the genus *Bifidobacterium* of the medium becomes $5 \times 10^6$ cfu/ml and that the bacterial count of the lactic acid bacterium becomes around $5 \times 10^6$ cfu/ml and that the bacteria are cultured at a temperature which is 3°C or more lower than the optimum culture temperature of the lactic acid bacterium or the like until the pH becomes around 4.4.

By the method of the present invention explained above, the culture of the present invention is obtained. The change in the acidity of the culture of the present invention is inhibited also during storage, and the viability of the lactic acid bacterium and/or the bacterium belonging to the genus *Bifidobacterium* contained in the culture is also high.

Specifically, the culture of the present invention is a culture in which the change in the acidity of the culture after storage at 10°C for 21 days is smaller than the change in the acidity of a culture obtained by culturing the lactic acid bacterium and/or the bacterium belonging to the genus *Bifidobacterium* at the optimum culture temperature of the lactic acid bacterium and/or the bacterium belonging to the genus *Bifidobacterium* after storage at 10°C for 21 days, preferably 1.40 to 1.45, and preferably in which, in addition to the above, the viable cell count of the culture before storage is $1.2 \times 10^9$ cfu/ml or more, preferably $1.2 \times 10^9$ cfu/ml to $2.0 \times 10^9$ cfu/ml and is 110% or more, preferably 110 to 130% of the viable cell count of a culture obtained by culturing the lactic acid bacterium and/or the bacterium belonging to the genus *Bifidobacterium* at the optimum culture temperature of the lactic acid bacterium and/or the bacterium belonging to the genus *Bifidobacterium* after storage at 10°C for 21 days.

Moreover, the culture of the present invention is a culture in which the acidity before storage is 4 to 12 and in which the change in the acidity after storage at 10°C for 21 days is smaller than 1.45, preferably 1.40 to 1.45. Preferably, in addition to the above, the culture of the present invention is a culture in which the viable cell count of the culture before storage is $1.2 \times 10^9$ cfu/ml or more, preferably $1.2 \times 10^9$ cfu/ml to $1.5 \times 10^9$ cfu/ml and in which the viability after storage at 10°C for 21 days is 80% or more, preferably 80 to 90%.

Furthermore, the culture of the present invention is a culture in which the viable cell count of the culture before storage is $1.2 \times 10^9$ cfu/ml or more, preferably $1.2 \times 10^9$ cfu/ml to $2.0 \times 10^9$ cfu/ml, and in which the viability after storage at 10°C for 21 days is 80% or more, preferably 80 to 90%.

In the present invention, the viable cell count, the change in the acidity and the viability are values measured by the methods described in the Examples.

The culture of the present invention explained above can be used as a fermented food or drink directly or after blending a food material such as a sweetener like syrup, sugar, a thickener, an emulsifier, a vitamin and a flavor by a conventionally known method depending on the need. Examples of the fermented food or drink include not only fermented milk specified by Ministerial Ordinance Concerning Compositional Standards, etc. for Milk and Milk Products but also beverages such as dairy lactic acid bacteria beverages and lactic acid bacteria beverages and those containing live bacteria such as kefir and yogurt. Examples of the form include hard type, soft type, plain type, sweetened type, fruit type, drink type, frozen type and the like.

The food materials which can be blended in the culture of the present invention are saccharides such as sucrose, glucose, fructose, palatinose, trehalose, lactose, xylose and maltose; sugar alcohols such as sorbitol, xylitol, erythritol, lactitol, Palatinit, reduced starch syrup and reduced maltose syrup; high-intensity sweeteners such as aspartame, thaumatin, sucralose, acesulfame K and stevia; thickeners (stabilizers) such as agar, gelatin, carrageenan, guar gum, xanthan gum, pectin, locust bean gum, gellan gum, carboxymethylcellulose, soybean polysaccharides and propylene glycol alginate; emulsifiers such as sucrose fatty acid esters, glycerol fatty acid esters, polyglycerol fatty acid esters, sorbitan fatty acid esters and lecithin; milk fat such as cream, butter and sour cream; acidulants such as citric acid, lactic acid, acetic acid, malic acid, tartaric acid and gluconic acid; vitamins such as vitamin A, vitamin B's, vitamin C and vitamin E's; minerals such as calcium, magnesium, zinc, iron and manganese and flavors such as yogurt flavor, berry flavor, orange flavor, Chinese quince flavor, perilla flavor, citrus flavor, apple flavor, mint flavor, grape flavor, apricot flavor, pear, custard cream, peach, melon, banana, tropical flavor, herbal flavor, black tea and coffee flavor.

As the culture of the present invention, the changes in the acidity of the fermented foods and drinks during storage are inhibited, and the viability of the lactic acid bacteria and/or the bacteria belonging to the genus *Bifidobacterium* contained in the fermented foods and drinks is high.

Examples

The present invention is explained in detail below referring to Examples, but the present invention is not limited to the Examples.

Example 1

Production of Cultures and Lactic Acid Bacteria Beverages:

To a medium obtained by dissolving 15 w/w% (hereinafter "%" in the medium compositions means "w/w%") defatted milk powder, 2.0% glucose and 4.7% fructose in water, the components shown in Table 1 were added, and the mixtures were sterilized at 100°C for 62 minutes. To the media, *Lactobacillus casei* YIT9029 (FERM BP-1366: optimum culture temperature of 37°C) starter was inoculated at 0.5% (initial bacterial count: $5.0 \times 10^6$ cfu/ml). Culturing was started at the temperatures shown in Table 1, and culturing was conducted to the acidity standard of 24.0. To 70 parts by mass of the obtained cultures, 30 parts by mass of a 3% soybean polysaccharide solution were mixed, and the mixtures were homogenized at 15 MPa. By mixing 35 parts by mass of the homogenized mixtures and 65 parts by mass of syrup (containing 14% sucrose), lactic acid bacteria beverages having a viable cell count of $1.2 \times 10^9$ cfu/ml or more and acidity of 5.5 to 6.8 were obtained (products 1 to 3). The lactic acid bacteria beverages were dispensed into optically transparent containers (made of polystyrene) each in a volume of 100 ml, and after storage at 10°C, the changes in the acidity and the viable cell counts (and the viability) were measured by the following methods. The results are also shown in Table 1.

[Table 1]

| | | | | Product 1 | Product 2 | Product 3 |
|---|---|---|---|---|---|---|
| Component | milk peptide[*1] | | | 0 | 0.1% | 0.1% |
| | oolong tea extract[*2] | | | 0.1% | 0.2% | 0.2% |
| | monoglyceryl fatty acid ester[*3] | | | 0.02% | 0.02% | 0.02% |
| Culture Temperature | | | | 35°C | 35°C | 30°C |
| Acidity | 0 | days | | 5.84 | 5.80 | 5.93 |
| | 7 | days | | 6.63 | 6.53 | 6.63 |
| | 14 | days | | 7.09 | 7.10 | 7.03 |
| | 21 | days | | 7.32 | 7.40 | 7.36 |
| | 28 | days | | 7.52 | 7.79 | 7.56 |
| | 35 | days | | 7.72 | 7.99 | 7.78 |
| Δ Acidity | 21 | days | | 1.48 | 1.60 | 1.43 |
| | 28 | days | | 1. 68 | 1.99 | 1.63 |
| | 35 | days | | 1.88 | 2.19 | 1.85 |
| Viable Cell Count | 0 | days | | 1.70E+09 | 1.83E+09 | 1.85E+09 |
| | 7 | days | | 1.55E+09 | 1.51E+09 | 1.65E+09 |
| | 21 | days | | 1.29E+09 | 1.42E+09 | 1.59E+09 |
| | 28 | days | | 1.32E+09 | 1.41E+09 | 1.44E+09 |

(continued)

|  |  |  | Product 1 | Product 2 | Product 3 |
|---|---|---|---|---|---|
| Viability | 21 | days | 75.9% | 77.6% | 86.0% |
|  | 28 | days | 77.6% | 77.0% | 77.8% |
| *1: CE90-GMM (manufactured by Nippon Shinyaku Co., Ltd. : derived from casein, average molecular weight of 640) *2: manufactured by Yakult Material Co., Ltd. *3: DIMODAN MO90/D (manufactured by Danisco Japan Ltd.), main fatty acid is oleic acid | | | | | |

<Method for Measuring Acidity>

The titer (unit: ml) derived by adding 40 g of ion-exchanged water to 9 g of the culture and conducting neutralization titration with 0.1N sodium hydroxide until the pH becomes 8.5.

<Method for Measuring Viable Cell Count>

Measurement was conducted using BCP medium (Eiken Chemical Co., Ltd.).

<Method for Calculating Viability>

$$\text{viability (\%) on day X} = (\text{viable cell count on day X}/\text{viable cell count on day 0}) \times 100$$

<Method for Calculating Change in Acidity>

$$\text{change in acidity (\%) on day X} = (\text{acidity on day X}/\text{acidity on day 0}) \times 100$$

With respect to product 3, which contained a culture obtained by culturing in a medium obtained by adding the milk peptide and the oolong tea extract to the basic medium at 30°C, which is 7°C lower than the optimum culture temperature of *Lactobacillus casei* YIT9029, the viable cell count before storage (day 0) was high. Moreover, the change in the acidity after storage for 21 days was small, and the viability was also high.

On the other hand, with respect to product 2, which contained a culture obtained by culturing in a medium obtained by adding the milk peptide and the oolong tea extract to the basic medium at 35°C, which is 2°C lower than the optimum culture temperature of *Lactobacillus casei* YIT9029, although the viable cell count before storage (day 0) was as high as that of product 3, the change in the acidity after storage for 21 days was larger, and the viability was lower than those of product 3.

Moreover, with respect to product 1, which contained a culture obtained by culturing in the basic medium at 35°C, which is 2°C lower than the optimum culture temperature of *Lactobacillus casei* YIT9029 of 37°C, because the culture was obtained by culturing in the basic medium, which did not contain the milk peptide or the oolong tea extract, the viable cell count before storage (day 0) was lower than that of product 2. However, the change in the acidity after storage for 21 days was smaller, and the viability was comparable.

The results of product 1 and product 2 show that the viable cell count before storage further increases and that the change in the acidity becomes larger, when *Lactobacillus casei* YIT9029 is cultured in a medium obtained by adding the milk peptide and the oolong tea extract to the basic medium at 37°C, which is the optimum culture temperature of

*Lactobacillus casei* YIT9029.

The results obtained show unexpected effects as follows. In the general case in which a milk peptide and an oolong tea extract are added to a medium at a temperature around the optimum culture temperature of the lactic acid bacterium or the like, the viable cell count before storage is high, but the change in the acidity is also large accordingly. However, when culturing is conducted at a temperature which is 3°C or more lower than the optimum culture temperature, which is not generally employed, the change in the acidity after storage is small, and the viability is high, although the viable cell count before storage is high.

Example 2

Production of Cultures and Lactic Acid Bacteria Beverages:

Product 4 and product 5 were obtained in the same manner as in the production of product 3 of Example 1 except that the amount of the milk peptide added to the basic medium was 0.025% or 0.2%.

The viable cell counts of the products before storage were $1.7 \times 10^9$ cfu/ml (product 4) and $1.7 \times 10^9$ cfu/ml (product 5), and the changes in the acidity and the viability after storage at 10°C for 21 days were comparable to those of product 3. It was found that the change in the acidity was smaller when the milk peptide content was 0.1 to 0.2% rather than 0.025%.

Example 3

Production of Culture and Lactic Acid Bacteria Beverage:

Product 6 was obtained in the same manner as in the production of product 3 of Example 1 except that the amount of the oolong tea extract, which is an auxiliary culture agent, added to the basic medium was 0.15%.

The viable cell count of the product before storage was $1.5 \times 10^9$ cfu/ml (product 6), and the change in the acidity and the viability after storage at 10°C for 21 days were comparable to those of product 3.

The results show that changing the amount of the oolong tea extract, which is an auxiliary culture agent, added to the basic medium does not affect the product.

Example 4

Production of Cultures and Lactic Acid Bacteria Beverages:

Product 7 and product 8 were obtained in the same manner as in the production of product 6 of Example 3 except that the kind of the milk peptide added to the basic medium was changed from CE90-GMM to MCH-30 (manufactured by Morinaga Milk Industry Co., Ltd.: derived from casein, average molecular weight of 230) or LF80GF-US (manufactured by Nippon Shinyaku Co., Ltd.: derived from whey, average molecular weight of 7800) .

The viable cell counts of the products before storage were $1.5 \times 10^9$ cfu/ml (product 7) and $1.6 \times 10^9$ cfu/ml (product 8), and the changes in the acidity and the viability after storage at 10°C for 21 days were comparable to those of product 3.

The results show that changing the kind of the milk peptide added to the basic medium does not affect the product.

Example 5

Production of Cultures and Lactic Acid Bacteria Beverages:

Product 9, product 10, product 11 and product 12 were obtained in the same manner as in the production of product 6 of Example 3 except that the culture temperature was 22°C, 24°C, 30°C or 34°C.

The viable cell counts of the products before storage were $1.7 \times 10^9$ cfu/ml (product 9), $1.7 \times 10^9$ cfu/ml (product 10), $1.8 \times 10^9$ cfu/ml (product 11) and $1.8 \times 10^9$ cfu/ml (product 12), and the changes in the acidity and the viability after storage at 10°C for 21 days were comparable to those of product 3.

The results show the following points . When the culture temperature is 3°C or more lower than the optimum culture temperature of *Lactobacillus casei* YIT9029 of 37°C, the viable cell count before storage is high. Moreover, the change in the acidity after storage for 21 days is small, and the viability is high. Within the temperature range, in particular, there is a tendency toward a higher viable cell count before storage when the culture temperature is higher, while there is a tendency toward a smaller change in the acidity when the culture temperature is lower. Moreover, the viability becomes high at around 30 to 34°C. Taking the viable cell count before storage, the change in the acidity after storage and the viability into consideration, the culture temperature is preferably 5°C or more, particularly preferably 5 to 15°C lower than the optimum culture temperature of

*Lactobacillus casei* YIT9029 of 37°C.

Example 6

Production of Cultures:

A basic medium was obtained by heat sterilizing a 9.7% defatted milk powder solution at 121°C for 15 minutes. *Bifidobacterium breve* YIT12272 (FERM BP-11320: optimum culture temperature of 37°C) starter was inoculated to the basic medium at 1% (initial bacterial count: $7.0\times10^6$ cfu/ml) and cultured at the temperatures shown in Table 2 until the pH became 4.9 to 5.0, and cultures 1 and 2 were thus obtained.

Moreover, cultures 3 to 6 were obtained in the same manner except that a milk peptide (CE90-GMM (manufactured by Nippon Shinyaku Co., Ltd.: derived from casein, average molecular weight of 640)) in the amounts shown in Table 2 was added to the basic medium.

[Table 2]

| | Culture 1 | Culture 2 | Culture 3 | Culture 4 | Culture 5 | Culture 6 |
|---|---|---|---|---|---|---|
| Milk peptide | not added | | 0.10% | | 0.30% | |
| Temperature | 30°C | 37°C | 30°C | 37°C | 30°C | 37°C |
| Culture period | 65.0 | 23.5 | 39.5 | 19.0 | 25.0 | 17.5 |
| pH | 4.92 | 4.95 | 4.99 | 4.97 | 4.98 | 4.94 |
| Viable cell count (cfu/ml) | $2.1\times10^8$ | $2.9\times10^8$ | $3.8\times10^8$ | $2.6\times10^8$ | $4.7\times10^8$ | $3.2\times10^8$ |

The results show that, not only when a lactic acid bacterium is used but also when a bacterium belonging to the genus *Bifidobacterium* is used, the viable cell count of the culture increases by culturing a bacterium belonging to the genus *Bifidobacterium* in a medium containing a milk peptide at 30°C, which is 7°C lower than the optimum temperature.

Example 7

Production of Cultures

Cultures 7 to 10 were obtained in the same manner as cultures 1, 2, 5 and 6 of Example 6 except that the basic medium was obtained by sterilizing a solution containing 11.5% defatted milk powder, 0.1% yeast extract (manufactured by BD), 0.03% L-cysteine hydrochloride (manufactured by Wako Pure Chemical Industries, Ltd.) and 0.2% calcium carbonate (manufactured by Kanto Chemical Co., Inc.) in a sealed container filled with nitrogen at 115°C for 15 minutes.

[Table 3]

| | Culture 7 | Culture 8 | Culture 9 | Culture 10 |
|---|---|---|---|---|
| Milk peptide | not added | | 0.30% | |
| Temperature | 30°C | 37°C | 30°C | 37°C |
| Culture period | 18.0 | 11.5 | 12.0 | 7.0 |
| pH | 4.71 | 4.76 | 4.75 | 4.77 |
| Viable cell count (cfu/ml) | $3.0\times10^9$ | $3.2\times10^9$ | $3.6\times10^9$ | $3.3\times10^9$ |

The results show that, also when a sufficiently high bacterial count is obtained by culturing in more suitable environment for a bacterium belonging to the genus *Bifidobacterium,* the viable cell count of culture 9, which was cultured at 30°C, which is 7°C lower than the optimum temperature, was higher than that of culture 10, which was cultured at the optimum temperature of 37°C.

Industrial Applicability

The method for producing a culture of the present invention and the culture produced by the method can be used for producing a fermented food or drink which has a high viable cell count at the time of the production and which can maintain the quality of the product also during storage.

**Claims**

1.  A method for producing a culture for obtaining a culture by culturing a lactic acid bacterium and/or a bacterium belonging to the genus *Bifidobacterium* in a milk peptide-containing medium which is **characterized in that** a culture temperature is 3°C or more lower than an optimum culture temperature of the lactic acid bacterium and/or the bacterium belonging to the genus *Bifidobacterium.*

2.  The method for producing a culture according to claim 1, wherein the medium further contains one or more kinds of auxiliary culture agent selected from the group consisting of a Chinese sweet tea extract, an oolong tea extract, a green tea extract, a black tea extract, a jasmine tea extract and oleic acid.

3.  The method for producing a culture according to claim 1 or 2, wherein the lactic acid bacterium is one or more kinds selected from the group consisting of a bacterium belonging to the genus *Lactobacillus,* a bacterium belonging to the genus *Lactococcus* and a bacterium belonging to the genus *Streptococcus.*

4.  The method for producing a culture according to claim 1 or 2, wherein the lactic acid bacterium and/or the bacterium belonging to the genus *Bifidobacterium* is one or more kinds selected from the group consisting of *Lactobacillus casei, Lactococcus lactis, Streptococcus thermophilus* and *Bifidobacterium breve.*

5.  The method for producing a culture according to claim 1 or 2, wherein the lactic acid bacterium and/or the bacterium belonging to the genus *Bifidobacterium* is one or more kinds selected from the group consisting of *Lactobacillus casei* YIT9029 (FERM BP-1366), *Lactococcus lactis* YIT2027 (FERM BP-6224), *Streptococcus thermophilus* YIT2021 (FERM BP-7537) and *Bifidobacterium breve* YIT12272 (FERM BP-11320).

6.  The method for producing a culture according to any one of claims 1 to 5, wherein the culture temperature is 5°C or more lower than the optimum culture temperature of the lactic acid bacterium and/or the bacterium belonging to the genus *Bifidobacterium.*

7.  The method for producing a culture according to any one of claims 1 to 6, wherein the change in the acidity of the culture after storage at 10°C for 21 days is smaller than the change in the acidity of a culture obtained by culturing the lactic acid bacterium and/or the bacterium belonging to the genus *Bifidobacterium* at the optimum culture temperature of the lactic acid bacterium and/or the bacterium belonging to the genus *Bifidobacterium* after storage at 10°C for 21 days.

8.  The method for producing a culture according to any one of claims 1 to 7, wherein the viable cell count of the culture before storage is $1.2 \times 10^9$ cfu/ml or more and is 110% or more of the viable cell count of a culture obtained by culturing the lactic acid bacterium and/or the bacterium belonging to the genus *Bifidobacterium* at the optimum culture temperature of the lactic acid bacterium and/or the bacterium belonging to the genus *Bifidobacterium* after storage at 10°C for 21 days.

9.  A culture produced by the method for producing a culture according to any one of claims 1 to 6 which is **characterized in that** the acidity of the culture before storage is 4 to 12 and that the change in the acidity after storage at 10°C for 21 days is smaller than 1.45.

10. The culture according to claim 9, wherein the viable cell count of the culture before storage is $1.2 \times 10^9$ cfu/ml or more, and the viability after storage at 10°C for 21 days is 80% or more.

11. A culture produced by the method for producing a culture according to any one of claims 1 to 6 which is **characterized in that** the viable cell count of the culture before storage is $1.2 \times 10^9$ cfu/ml or more and that the viability after storage at 10°C for 21 days is 80% or more.

12. A fermented food or drink **characterized by** containing a culture produced by the production method according to any one of claims 1 to 8.

13. A fermented food or drink **characterized by** containing the culture according to any one of claims 9 to 11.

## INTERNATIONAL SEARCH REPORT

| | International application No. |
|---|---|
| | PCT/JP2020/003848 |

**A. CLASSIFICATION OF SUBJECT MATTER**
Int.Cl. A23C9/123(2006.01)i, A23C9/13(2006.01)i, C12N1/20(2006.01)i
FI: C12N1/20A, A23C9/123, A23C9/13

According to International Patent Classification (IPC) or to both national classification and IPC

**B. FIELDS SEARCHED**

Minimum documentation searched (classification system followed by classification symbols)
Int.Cl. A23C9/123, A23C9/13, C12N1/20

Documentation searched other than minimum documentation to the extent that such documents are included in the fields searched
Published examined utility model applications of Japan       1922-1996
Published unexamined utility model applications of Japan     1971-2020
Registered utility model specifications of Japan             1996-2020
Published registered utility model applications of Japan     1994-2020

Electronic data base consulted during the international search (name of data base and, where practicable, search terms used)
JSTPlus/JMEDPlus/JST7580(JDreamIII),
CAplus/MEDLINE/EMBASE/BIOSIS/WPIDS/WPIX(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | VANINGELGEM, F. et al., Fermentation conditions affecting the bacterial growth and exopolysaccharide production by Streptococcus thermophilus ST 111 in milk-based medium, Journal of Applied Microbiology, 2004, vol. 97, pp. 1257-1273, abstract, table 5 | 1, 3-13<br>2 |
| X<br>Y | PRASANNA, P. H. P. et al., Effect of dairy-based protein sources and temperature on growth acidification and exopolysaccharide production of Bifidobacterium strains in skim milk, Food Research International, 2012, vol. 47, pp. 6-12, abstract, fig. 3, 4 | 1, 3-13<br>2 |
| Y<br>A | WO 2006/126476 A1 (YAKULT HONSHA KK) 30.11.2006 (2006-11-30), claims, paragraphs [0010], [0036] | 2<br>1, 3-13 |

☐ Further documents are listed in the continuation of Box C.    ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 09.03.2020 | 07.04.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

| International application No. |
|---|
| PCT/JP2020/003848 |

```
WO 2006/126476 A1  30.11.2006    US 2008/0292751 A1
                                  claims, paragraphs [0019], [0047]
                                  EP 1884566 A1
```

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013133313 A
- JP 2001178413 A
- JP 2016174589 A